# DEMANDE DE BREVET EUROPEEN

(11) **EP 4 789 567 A1**
(43) Date de publication de la demande: **12.08.2026**
(21) Numéro de dépôt: 26156525.3
(22) Date de dépôt: 05.02.2026
(51) Int. Cl.: A41C 3/00, A41D 27/20, A61M 25/02, A61M 5/142

(54) **ACCESSOIRE VESTIMENTAIRE, NOTAMMENT POUR LE BUSTE, DE TYPE SOUTIEN-GORGE OU BRASSIÈRE, COMPRENANT UNE POCHE DE RECEPTION D'UN BOÎTIER D'UN DISPOSITIF D'INJECTION DE LIQUIDE**

(30) Priorité: 05.02.2025 FR 2501168
(71) Demandeur: Drevaux, Caroline, 77370 Nangis (FR)
(72) Inventeur: Drevaux, Caroline, 77370 Nangis (FR)
(74) Mandataire: Lavoix

(57) **Abrégé**

La présente invention concerne un accessoire vestimentaire (10) de type soutien-gorge ou brassière comprenant un dispositif de maintien (12) sur le corps (15) et une poche (20) de réception d'un boîtier (25) d'un dispositif médical (30) d'injection de liquide comprenant un réservoir (35) de liquide connecté à une tubulure (40). La poche (20) est solidaire du dispositif de maintien (12) et présente une paroi latérale (85) s'étendant selon un axe principal (Z2) entre un fond (90) et une ouverture supérieure (95) configurée pour permettre l'extraction du boîtier (25) hors de la poche (20) par translation selon l'axe principal (Z2) du fond (90) vers l'ouverture supérieure (95). Le fond (90) comprend une ouverture inférieure (110) et un dispositif de fermeture principal (115) présentant une position ouverte permettant l'insertion du boîtier (25) par translation de l'ouverture inférieure (110) vers l'ouverture supérieure (95) et une position fermée empêchant le passage du boîtier (25) au travers de l'ouverture inférieure (110) tout en permettant la translation de la tubulure (40).

## Description

La présente invention concerne un accessoire vestimentaire, notamment pour le buste, comprenant une poche de réception d'un boîtier d'un dispositif médical d'injection de liquide, le boîtier comprenant un réservoir de liquide connecté à une tubulure du dispositif médical s'étendant hors du boîtier.

Les dispositifs médicaux d'injection de liquide portables sont utilisés dans le traitement de nombreuses maladies, de manière à assurer une injection personnalisée, itérative et automatisée d'un ou plusieurs liquides thérapeutiques dans le corps d'un patient.

En particulier, les pompes à insuline sont utilisées couramment comme alternative au traitement par multi-injections avec des stylos à insuline.

Les pompes à insuline tubulaires, et plus généralement les pompes à médicaments tubulaires, comportent un boîtier comprenant un réservoir de médicament relié à une tubulure.

La tubulure est reliée d'une part au réservoir et d'autre part à une canule à insérer dans le corps du patient, par exemple pour une injection sous-cutanée du médicament.

Le boîtier comprend par ailleurs une pompe, une batterie et des moyens électroniques programmables de contrôle de la pompe.

La pompe à médicaments tubulaire peut de manière connue être maintenue sur le corps du patient au moyen d'un accessoire vestimentaire, tel qu'une ceinture, une culotte ou un tee-shirt, sur lequel une poche de réception du boîtier est formée.

Les documents FR 2 869 505, WO 2015/198060 et WO 2016/054386 décrivent de tels accessoires vestimentaires.

La poche de réception comprend dans ces documents une ouverture, éventuellement fermée par un rabat, pour l'insertion et l'extraction du boîtier et de la tubulure.

Dans tous les modes de réalisation décrits dans ces documents, la tubulure doit être insérée et extraite dans/hors de la poche en même temps que le boîtier et nécessite un soin particulier lors de la manipulation du boîtier.

De tels poches ne permettent donc pas une extraction temporaire aisée et sure du boîtier hors de la poche pour une interaction temporaire du patient avec une interface utilisateur, par exemple pour consultation d'un écran de contrôle ou pour provoquer manuellement une injection de médicament.

Notamment, la tubulure est coudée ou repliée lors de son insertion dans la poche, et le cas échéant, contrainte par l'éventuel rabat de fermeture de la poche, de sorte que les risques d'arrachage, de blocage ou de pincement de la tubulure lors de l'extraction ou l'insertion du boîtier dans la poche sont importants.

Un but de l'invention est alors de proposer un accessoire vestimentaire, notamment pour le buste, comprenant une poche de réception d'un boîtier d'un dispositif médical d'injection de liquide comprenant un réservoir de liquide connecté à une tubulure, permettant une manipulation plus aisée et plus sure du boîtier, ainsi qu'un port confortable du boîtier et si possible discret.

A cet effet, l'invention a pour objet un accessoire vestimentaire comprenant un dispositif de maintien sur le corps, notamment sur le buste, d'un utilisateur et une poche de réception d'un boîtier d'un dispositif médical d'injection de liquide, le boîtier comprenant un réservoir de liquide connecté à une tubulure du dispositif médical s'étendant hors du boîtier,
- la poche étant solidaire du dispositif de maintien,
- la poche présentant une paroi latérale s'étendant selon un axe principal entre un fond et une ouverture supérieure destinée à être positionné plus haut que le fond selon un axe longitudinal d'un utilisateur orienté des pieds vers la tête de cet utilisateur, l'ouverture supérieure étant configurée pour permettre l'extraction du boîtier hors de la poche par translation selon l'axe principal du fond vers l'ouverture supérieure,
- l'accessoire vestimentaire étant caractérisé en ce que le fond comprend une ouverture inférieure et un dispositif de fermeture principal de l'ouverture inférieure présentant :
- au moins une position ouverte, permettant l'insertion du boîtier dans la poche par translation selon l'axe principal de l'ouverture inférieur vers l'ouverture supérieure, et
- au moins une position fermée, empêchant le passage du boîtier au travers de l'ouverture inférieure tout en permettant la translation de la tubulure selon l'axe principal.

Lorsque le dispositif de fermeture principal est en position ouverte, l'ouverture inférieure permet l'insertion du boîtier dans la poche par une translation selon l'axe principal dans le sens de l'ouverture inférieure vers l'ouverture supérieure, c'est-à-dire des pieds vers la tête d'un patient porteur de l'accessoire vestimentaire, y compris si la tubulure est déjà connectée d'une part au réservoir par l'intermédiaire d'un connecteur et d'autre part à une canule déjà inserée dans un site d'insertion du corps du patient.

La position ouverte du dispositif de fermeture principal permet en particulier de positionner le connecteur de la tubulure du côté du fond de la poche.

Le site d'insertion peut alors être choisi sur le patient porteur de l'accessoire vestimentaire en-dessous du fond de la poche selon l'axe longitudinal du patient sans risque de repliement, de pincement ou d'arrachage de la tubulure.

Lorsque le dispositif de fermeture principal est en position fermée, le boîtier ne peut pas sortir de la poche par l'ouverture inférieure. En revanche, la tubulure peut coulisser au travers de l'ouverture inférieure selon l'axe principal, mais ses déplacements dans un plan transversal orthogonal à l'axe principal sont limités par la présence du dispositif de fermeture principal. La tubulure est donc guidée lors d'une éventuelle extraction du boîtier hors de la poche à travers l'ouverture supérieure, par translation du boîtier selon l'axe principal dans le sens de l'ouverture inférieure vers l'ouverture supérieure, c'est-à-dire des pieds vers la tête du patient porteur de l'accessoire vestimentaire.

Le patient peut donc aisément consulter le boîtier en le rapprochant de ses yeux, exercer optionnellement une action manuelle sur ce boîtier, puis repositionner le boîtier dans la poche, sans que la tubulure ne risque de s'arracher, de se replier ou de se bloquer.

Le choix des dimensions du dispositif de fermeture principal, notamment son extension dans le plan transversal, permet d'assurer un maintien selon la direction transversale peut plus ou moins lâche.

Dans tous les cas, l'ouverture inférieure permet d'assurer un meilleur contrôle de la position de la tubulure dans le plan transversal que les accessoires vestimentaires de l'art antérieur.

Suivant d'autres aspects avantageux de l'invention, l'accessoire vestimentaire comprend une ou plusieurs des caractéristiques suivantes, prises isolément ou suivant toutes les combinaisons techniquement possibles :
- l'ouverture supérieure et l'ouverture inférieure sont espacées selon l'axe principal d'une distance supérieure ou égale à 90% d'une hauteur du boîtier du dispositif médical d'injection de liquide entre une face du boîtier sur laquelle est montée un connecteur de la tubulure et une face opposée du boîtier ;
- l'accessoire vestimentaire comprend en outre un dispositif de fermeture auxiliaire de l'ouverture supérieure présentant :
   * au moins une position ouverte, permettant l'extraction du boîtier hors de la poche par translation selon l'axe principal du fond vers l'ouverture supérieure, et
   * au moins une position fermée, empêchant le passage du boîtier au travers de l'ouverture supérieure ;
- le dispositif de fermeture principal et/ou, le cas échéant, le dispositif de fermeture auxiliaire est choisi parmi un fermoir à pression, un fermoir magnétique, un fermoir à agrafe et une fermeture à glissière ;
- le dispositif de maintien comprend une bande sous-poitrine de laquelle le fond de la poche est solidaire ;
- la poche s'étend au-dessus de la bande sous-poitrine dans une portion entre-seins ;
- l'accessoire vestimentaire est de type soutien-gorge ou brassière ;
- l'accessoire vestimentaire est de type soutien-gorge, et une première face de la paroi latérale de la poche s'étend à partir de la bande sous-poitrine, deux bonnets étant placés de part et d'autre de la première face et liés à la bande sous-poitrine, une deuxième face de la poche, destinée à être en contact avec l'utilisateur, étant rapportée sur la première face ;
- la première face s'étend à partir de ou est formée dans une pièce sous-poitrine avant interne de la bande sous-poitrine rapportée sur une pièce sous-poitrine avant externe de la bande sous-poitrine et l'accessoire vestimentaire comprend un élastique sous-poitrine lié à la pièce sous-poitrine avant externe s'étendant sur toute la longueur de la bande sous-poitrine perpendiculairement à l'axe principal, et
- l'accessoire vestimentaire est de type brassière, et la poche est rapportée sur une face interne d'un devant d'une brassière conventionnelle.

L'invention apparaîtra plus clairement à la lecture de la description qui va suivre, donnée uniquement à titre d'exemple non limitatif, et faite en référence aux dessins dans lesquels :
la figure 1 représente schématiquement un dispositif médical d'injection de liquide tubulaire ;
la figure 2 représente des sites d'insertion possibles de la canule du dispositif médical de la figure 1, notamment dans le cas où ce dispositif médical est une pompe à insuline, sur un utilisateur vu de face (partie A) et de dos (partie B) ;
la figure 3 représente un accessoire vestimentaire selon l'invention selon un premier mode de réalisation, le boîtier du dispositif médical de la figure 1 étant en cours d'insertion dans la poche au travers de l'ouverture inférieure, le dispositif de fermeture principal étant dans sa position ouverte ;
la figure 4 représente l'accessoire vestimentaire de la figure 3, le dispositif de fermeture principal étant dans sa position fermée et le boîtier du dispositif médical étant en place dans la poche ;
la figure 5 représente l'accessoire vestimentaire de la figure 3, le dispositif de fermeture principal étant dans sa position fermée, la tubulure étant en place dans la poche et le boîtier du dispositif médical ayant été extrait par l'ouverture supérieure ;
la figure 6 représente partiellement un patron de l'accessoire vestimentaire des figures 3 à 5 ;
la figure 7 représente un accessoire vestimentaire selon l'invention selon un deuxième mode de réalisation ;
la figure 8 représente la poche de l'accessoire vestimentaire des figures 3 à 7 seule, avec sur la partie A, le dispositif de fermeture principal en position fermée et le dispositif de fermeture auxiliaire en position ouverte, et sur la partie B, le dispositif de fermeture principal et le dispositif de fermeture auxiliaire tous deux en position ouverte ; et
la figure 9 représente le résultat de trois des étapes d'un procédé de fabrication de l'accessoire vestimentaire des figure 3 à 5, à savoir les étapes b) (partie B) ; c) (partie A) et j) (partie C).

L'invention porte sur un accessoire vestimentaire 10 comprenant un dispositif de maintien 12 sur le corps 15, notamment sur le buste 17, d'un utilisateur et une poche 20 de réception d'un boîtier 25 d'un dispositif médical 30 d'injection de liquide, la poche 20 étant solidaire du dispositif de maintien 12.

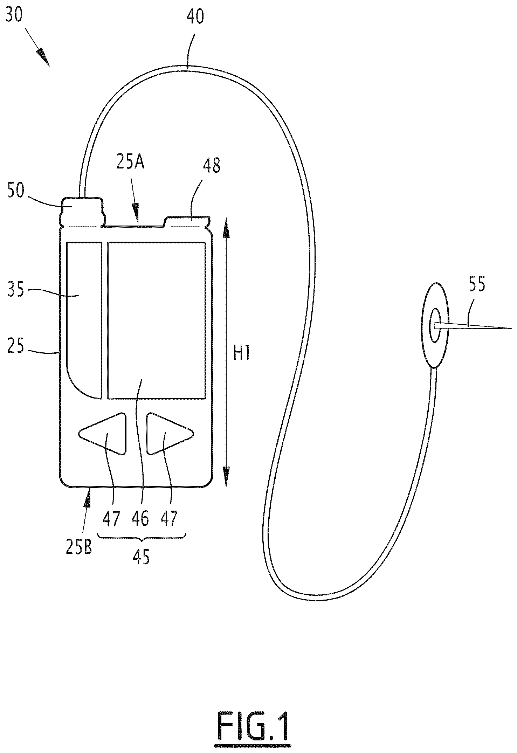

Le dispositif médical 30 d'injection de liquide est décrit en référence à la figure 1.

Le boîtier 25 comprend un réservoir 35 de liquide connecté à une tubulure 40 du dispositif médical 30 ainsi qu'une interface utilisateur 45.

L'interface utilisateur 45 comprend par exemple un écran 46 et/ou un ou plusieurs boutons 47 de commande du dispositif médical 30, tels qu'un bouton marche/arrêt ou un bouton de commande d'une injection d'une dose de liquide.

Le réservoir 35 est destiné à recevoir un liquide comprenant un ou plusieurs médicaments, par exemple de l'insuline.

De manière connue, le dispositif médical 30 comprend une pompe (non représentée) permettant de faire circuler le liquide depuis le réservoir 35 vers la tubulure 40, des moyens électroniques programmables de contrôle de la pompe (non représentés) et une batterie ou une pile 48.

La tubulure 40 s'étend entre un connecteur de tubulure 50, positionné sur une première face 25A du boîtier 25 et une canule d'injection 55.

La canule d'injection 55 est destinée à être insérée dans le corps 15 de l'utilisateur au niveau d'un site d'insertion 60.

La canule d'injection 55 permet ainsi l'injection du liquide contenu dans le réservoir 35 par voie sous-cutanée, intramusculaire ou intraveineuse.

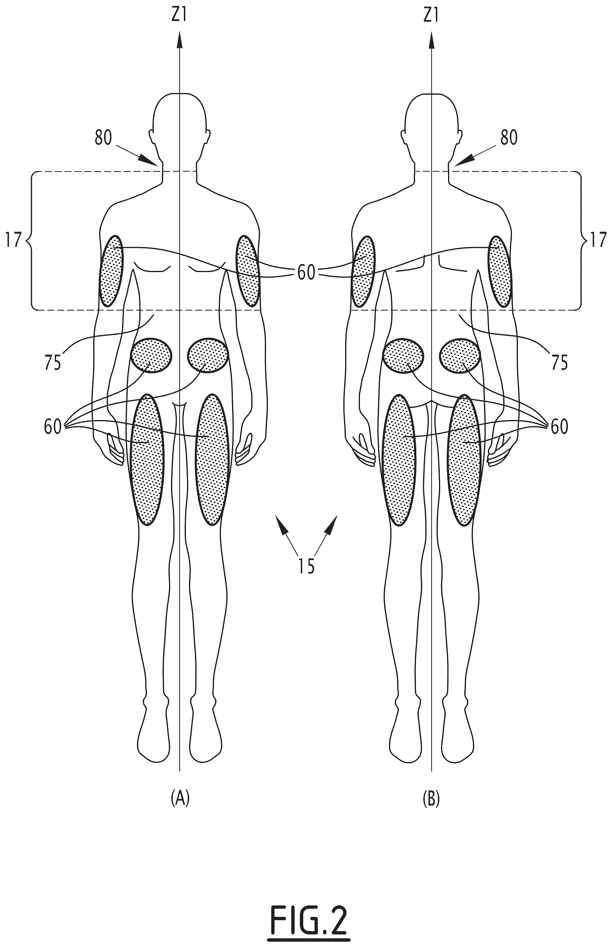

La figure 2 représente des sites d'insertion 60 sous-cutanée privilégiés dans le cas où le dispositif médical 30 est une pompe à insuline. Le site d'insertion 60 est avantageusement situé dans l'abdomen, de manière à assurer une diffusion de l'insuline particulièrement rapide. Alternativement, le site d'insertion 60 est par exemple situé dans une fesse, une cuisse ou sur un bras de l'utilisateur.

L'accessoire vestimentaire 10 est configuré pour permettre de maintenir le boîtier 25 à proximité d'une position souhaitée sur le corps 15 de l'utilisateur, en particulier une fois la canule d'injection 55 en place dans son site d'insertion 60.

A cette fin, l'accessoire vestimentaire 10 comprend le dispositif de maintien 12.

Le dispositif de maintien 12 comprend par exemple au moins une bande de maintien, telle qu'une bande sous-poitrine 65 ou une ceinture, et/ou au moins une bretelle 70 et/ou au moins une tête de manche.

L'accessoire vestimentaire 10 est notamment destiné à être porté sur le buste 17 de l'utilisateur, c'est-à-dire entre la taille 75 et le cou 80 de l'utilisateur. Cette disposition permet de positionner dans une majorité de cas le connecteur de tubulure 50 au-dessus du site d'insertion 60 selon l'axe longitudinal Z1 de l'utilisateur orienté des pieds vers la tête, comme représenté sur la figure 2, en particulier dans les cas où le site d'insertion 60 est choisi sur l'abdomen ou sur les cuisses de l'utilisateur.

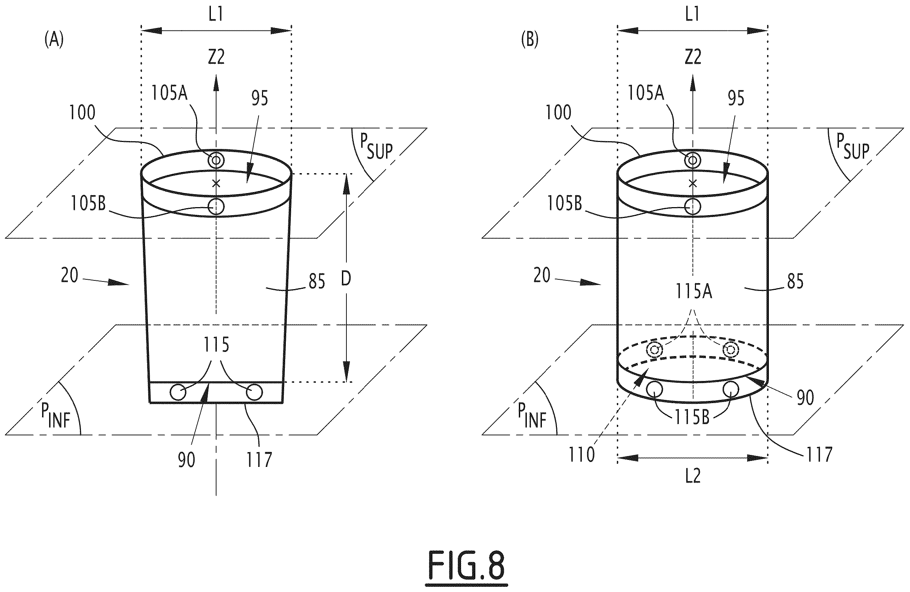

Le dispositif de maintien 12 est solidaire de la poche 20 de réception du boîtier 25. La poche 20 est décrite en référence à la figure 8.

La poche 20 présente une paroi latérale 85 s'étendant selon un axe principal Z2 entre un fond 90 et une ouverture supérieure 95.

L'axe principal Z2 de la poche 20 est de préférence positionné substantiellement parallèlement à l'axe longitudinal Z1 de l'utilisateur lorsque celui-ci se tient debout et immobile.

L'homme du métier comprendra à la lecture de la présente description que l'effet technique de l'invention peut être obtenu avec une tolérance sur l'écart angulaire entre les directions de l'axe principal Z2 de la poche 20 et l'axe longitudinal Z1 est assez grande.

L'ouverture supérieure 95 est par exemple formée au niveau d'un bord supérieur 100 de la poche 20, comme visible sur la figure 8, ou à proximité du bord supérieur 100.

La paroi latérale 85 délimite entre le fond 90 et le bord supérieur 100 un volume de réception suffisant pour la réception du boîtier 25, compte tenu d'une éventuelle élasticité du matériau dans lequel la poche 20, et en particulier la paroi latérale 85 et le fond 90, est formée.

En particulier, lorsque le boîtier 25 est positionné dans la poche 20, le fond 90 et le bord supérieur 100 sont espacés l'un de l'autre selon l'axe principal Z2 d'une distance D qui peut être au moins égale à une hauteur H1 du boîtier 25 entre la première face 25A et une deuxième face 25B du boîtier 25 opposée à la première face 25, éventuellement compte tenu d'une hauteur du connecteur de tubulure 50 selon cette direction.

La poche 20 est notamment formée dans un ou plusieurs matériaux textiles adaptés pour un usage vestimentaire, présentant optionnellement une élasticité choisie par rapport au poids du boîtier 25 et/ou au maintien souhaité et/ou à l'usage de l'accessoire vestimentaire 10.

L'ouverture supérieure 95 est destinée à être positionné plus haut que le fond 90 selon l'axe longitudinal Z1 de l'utilisateur orienté des pieds vers la tête de cet utilisateur. Autrement dit, l'ouverture supérieure 95 est destinée à être positionnée plus proche de la tête et plus loin des pieds de l'utilisateur que le fond 90.

L'ouverture supérieure 95 est configurée pour permettre l'extraction du boîtier 25 hors de la poche 20 par translation selon l'axe principal Z2 du fond 90 vers l'ouverture supérieure 95.

L'ouverture supérieure 95 s'étend donc selon au moins une direction d'un plan transversal supérieur P_{SUP} orthogonal à l'axe principal Z2 sur une première largeur L1 suffisante pour permettre l'extraction du boîtier 25 hors de la poche 20.

L'homme du métier comprendra que la première largeur L1 est choisie en fonction de l'élasticité d'un matériau dans lequel la poche 20 - et en particulier sa paroi latérale 85 et son bord supérieur 100 - est formée, et des superficies des première et deuxième faces 25A et 25B du boîtier 25.

L'ouverture supérieure 95 est donc configurée pour permettre l'extraction du boîtier 25 hors de la poche 20 par translation des pieds vers la tête de l'utilisateur lorsque l'axe principal Z2 est parallèle à l'axe longitudinal Z1.

Cette disposition permet à l'utilisateur de rapprocher aisément le boîtier 25 de ses yeux pour une interaction avec l'interface utilisateur 45, tout en laissant l'accessoire vestimentaire 10 en place.

La poche 20 comprend optionnellement un dispositif de fermeture auxiliaire 105 de l'ouverture supérieure 95.

Le dispositif de fermeture auxiliaire 105 présente :
- au moins une position ouverte, visible sur les figures 8A et 8B, permettant l'extraction du boîtier 25 hors de la poche 20 par sa translation selon l'axe principal Z2 du fond 90 vers l'ouverture supérieure 95, et
- au moins une position fermée empêchant le passage du boîtier 25 au travers de l'ouverture supérieure 95.

Cette disposition permet notamment d'éviter que le boîtier 25 ne risque de tomber de manière involontaire hors de la poche 20 lorsque l'utilisateur bouge, par exemple se penche, sans pour autant empêcher l'interaction de l'utilisateur avec l'interface utilisateur 45 lorsqu'il le souhaite.

Le dispositif de fermeture auxiliaire 105 est par exemple choisi parmi un fermoir à pression, un fermoir magnétique, un fermoir à agrafe et une fermeture à glissière.

De manière générale, le dispositif de fermeture auxiliaire 105 peut être choisi de manière à permettre l'ouverture et la fermeture répétée de l'ouverture supérieure 95 pendant une durée de vie satisfaisante de l'accessoire vestimentaire 10, tout en assurant une fermeture suffisamment robuste de l'ouverture supérieure 95 pour empêcher la chute du boîtier 25 lors des mouvements de l'utilisateur.

Cette disposition permet l'ouverture et la fermeture facile, fiable et discrète du dispositif de fermeture auxiliaire 105 par l'utilisateur, sans qu'il ne soit nécessaire d'ôter ou de modifier la position du dispositif de maintien 12.

Dans l'exemple de la figure 8, le dispositif de fermeture auxiliaire 105 comprend une partie mâle 105A et une partie femelle 105B d'un fermoir à pression.

La poche 20 est solidaire du dispositif de maintien 12 par exemple par couture d'au moins une fraction de la paroi latérale 85 et/ou du fond 90 et/ou du bord supérieur 100 sur le dispositif de maintien 12.

Le fond 90 comprend une ouverture inférieure 110 et un dispositif de fermeture principal 115 de l'ouverture inférieure 110.

L'ouverture inférieure 110 est par exemple formée au niveau d'un bord inférieur 117 de la poche 20, comme visible sur la figure 8, ou à proximité du bord inférieur 117.

Le dispositif de fermeture principal 115 présente :
- au moins une position ouverte, permettant l'insertion du boîtier 25 dans la poche 20 par translation selon l'axe principal Z2 de l'ouverture inférieure 110 vers l'ouverture supérieure 95, et
- au moins une position fermée, empêchant le passage du boîtier 25 au travers de l'ouverture inférieure 110 tout en permettant la translation de la tubulure 40 selon l'axe principal Z2.

L'ouverture inférieure 110 s'étend donc selon au moins une direction d'un plan transversal inférieur P_{INF} orthogonal à l'axe principal Z2 sur une deuxième largeur L2 suffisante pour permettre l'insertion du boîtier 25 dans la poche 20.

L'homme du métier comprendra que la deuxième largeur L2 est choisie en fonction de l'élasticité d'un matériau dans lequel la poche 20, et en particulier la paroi latérale 85 et le fond 90, est formée et des superficies des première et deuxième faces 25A et 25B du boîtier 25.

La deuxième largeur L2 est par exemple égale à la première largeur L1.

L'ouverture inférieure 110 est donc configurée pour permettre l'insertion du boîtier 25 dans la poche 20 par translation des pieds vers la tête de l'utilisateur lorsque l'axe principal Z2 est parallèle à l'axe longitudinal Z1.

Dans un mode de réalisation particulier, l'ouverture supérieure 95 et l'ouverture inférieure 110 sont espacées selon l'axe principal Z2 d'une distance supérieure ou égale à 90%, voire 95% ou 99% de la hauteur H1 du boîtier 25 entre la face 25A sur laquelle est montée le connecteur de tubulure 50 et la face 25B opposée.

Cette disposition permet une insertion et une extraction du boîtier 25 dans/hors de la poche 20 particulièrement aisée.

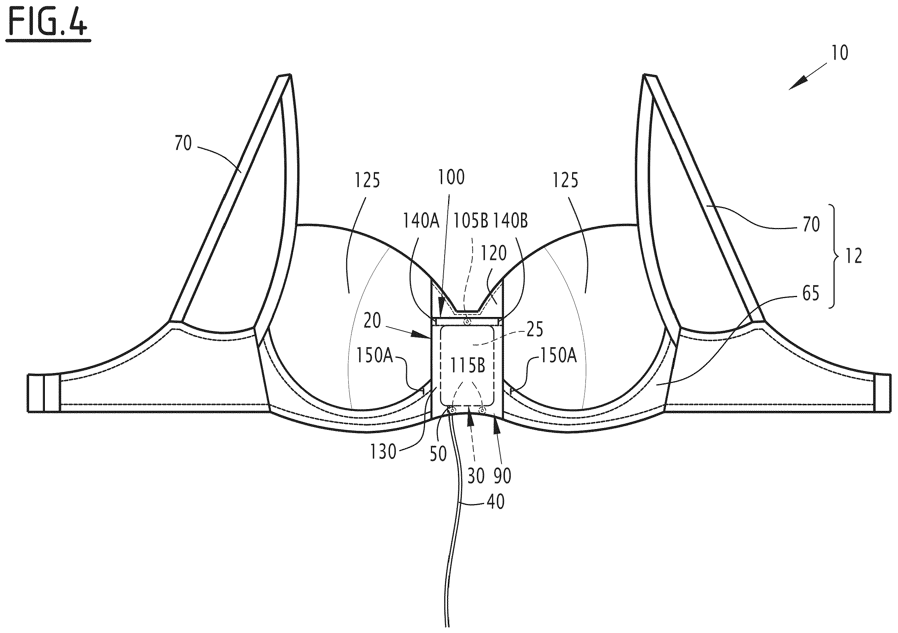

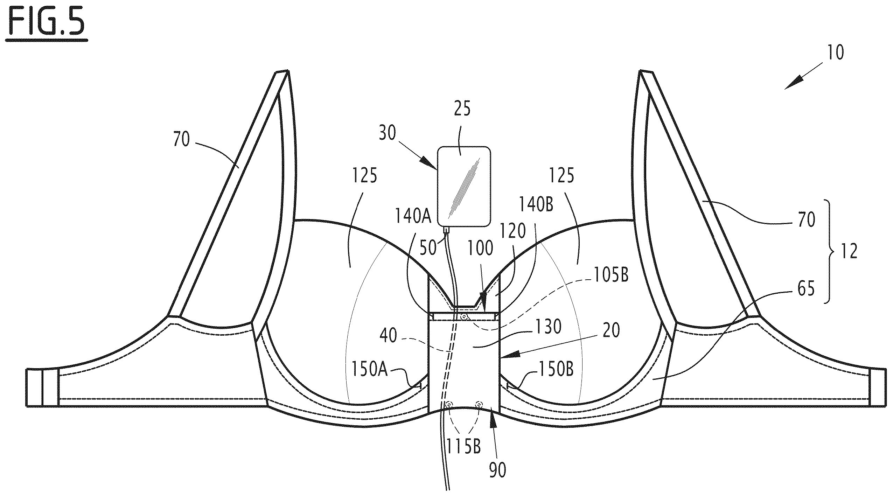

Le dispositif de fermeture principal 115 dans la position fermée obture une première fraction de l'ouverture inférieure 110 suffisante pour empêcher le passage de la première face 25A du boîtier 25 au travers de l'ouverture inférieure 110, tout en laissant ouverte une deuxième fraction de l'ouverture inférieure 110 suffisante pour que la tubulure 40 puisse coulisser au travers de cette deuxième fraction, comme visible sur les figures 4 et 5.

On comprend donc que l'ouverture inférieure 110 permet l'insertion du boîtier 25 par le fond 90 de la poche 20, par un mouvement de translation des pieds vers la tête de l'utilisateur lorsque le dispositif de fermeture principal 115 est ouvert, et le maintien du boîtier 25 dans la poche 20 lorsque le dispositif de fermeture principal 115 est fermé. L'ouverture supérieure 95 permet simultanément, comme on l'a vu précédemment, l'extraction du boîtier 25 par le haut de la poche 20, par un mouvement de translation des pieds vers la tête de l'utilisateur pour une interaction avec l'interface utilisateur 45. Ces mouvements du boîtier 25 sont possibles sans repliement ou pincement de la tubulure 40 y compris lorsque le site d'insertion 60 est situé sous le fond 90.

La position fermée du dispositif de fermeture principal 115 permet un guidage de la tubulure 40 en translation selon une direction parallèle à l'axe principal Z2 plus ou moins ferme, en restreignant la latitude de déplacement de la tubulure 40 dans le plan transversal inférieur P_{INF}, ce qui permet de diminuer de manière particulièrement efficace les risques de repliement, de pincement ou d'arrachage de la tubulure 40.

Le dispositif de fermeture principal 115 est par exemple choisi parmi un fermoir à pression, un fermoir magnétique, un fermoir à agrafe et une fermeture à glissière.

De manière générale, le dispositif de fermeture principal 115 peut être choisi de manière à permettre l'ouverture et la fermeture répétée de l'ouverture inférieure 110 pendant une durée de vie satisfaisante de l'accessoire vestimentaire 10, tout en assurant une fermeture suffisamment robuste de l'ouverture inférieure 110 pour empêcher la chute du boîtier 25 en position fermée.

Cette disposition permet l'ouverture et la fermeture facile et discrète du dispositif de fermeture principal 115 par l'utilisateur, sans qu'il ne soit nécessaire d'ôter ou de modifier la position du dispositif de maintien 12.

Dans l'exemple de la figure 8, le dispositif de fermeture principal 115 est un fermoir à pression comportant deux parties mâles 115A et deux parties femelle 115B d'un fermoir à pression.

Dans ce cas, chacun des trois espaces de l'ouverture inférieure 110 laissés libres lorsque les deux parties du fermoir à pression sont fermées peut être utilisé pour guider la tubulure 40 en translation selon l'axe principal Z2.

On comprend à la lumière de cet exemple que le choix des dimensions du dispositif de fermeture principal 115 confère à l'accessoire vestimentaire selon l'invention une certaine souplesse d'adaptation à différents dispositifs médicaux 30, pour lesquels le connecteur de tubulure 50 serait notamment placé différemment sur la première face 25A.

Le choix de l'élasticité du ou des matériaux dans lequel est formée la poche 20 accroit cette souplesse d'adaptation, en permettant l'insertion de boîtiers 25 dans une gamme de dimensions prédéterminées.

L'accessoire vestimentaire 10 est de préférence destiné à être porté sur le buste 17 de l'utilisateur.

La poche 20 est alors de préférence placée à l'intérieur de l'accessoire vestimentaire 10, de manière à être dissimulée. Cette disposition permet à l'utilisateur de porter le dispositif médical 30 de manière particulièrement discrète.

Dans ce cas, le dispositif de maintien 12 comprend de préférence une bande sous-poitrine 65 de laquelle le fond 90 de la poche 20 est solidaire. Cette disposition permet de maintenir de manière particulièrement précise la position du boîtier 25 lorsqu'il est dans la poche.

La poche 20 s'étend alors avantageusement au-dessus de la bande sous-poitrine 65, dans une portion entre-seins de l'accessoire vestimentaire 10. Cette disposition permet de dissimuler le boîtier 25 particulièrement efficacement, l'épaisseur des seins masquant la surépaisseur formée par le boîtier 25 lorsqu'il est dans la poche 20.

Pour mettre en place le boîtier 25 du dispositif médical 30 dans l'accessoire vestimentaire 10, notamment lorsque la canule 55 est en place dans le site d'insertion 60, il suffit d'insérer le boîtier 25 dans la poche 20 par l'ouverture inférieure 110, dispositif de fermeture principal 115 ouvert, puis de placer le dispositif de fermeture principal 115 en position fermée.

Le cas échéant, le dispositif de fermeture auxiliaire 105 est placé en position fermée, de préférence avant l'insertion du boîtier 25.

Pour extraire temporairement le boîtier 25 hors de la poche 20, il suffit le cas échéant de placer le dispositif de fermeture auxiliaire 105 en position ouverte, et de translater le boîtier 25 au travers de l'ouverture supérieure 95 en tirant sur le boîtier 25 vers le haut. La tubulure 40 coulissera naturellement à travers le dispositif de fermeture principal 115 en position fermée.

Pour replacer le boîtier 25 dans la poche 20, il suffit de l'insérer à nouveau dans la poche en le translatant au travers de l'ouverture supérieure 95 vers le fond 90, et si besoin de tirer légèrement sur la tubulure 40 en-dessous du fond 90 pour l'aider à coulisser vers le bas de manière à s'assurer qu'aucun coude ou repliement de la tubulure 40 ne se forme.

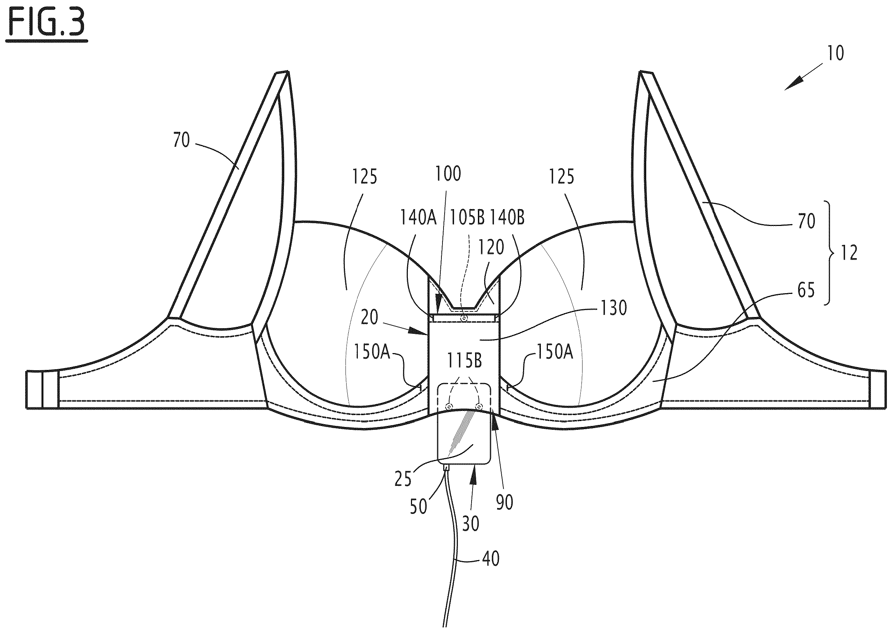

L'accessoire vestimentaire 10 selon un premier mode de réalisation va maintenant être décrit de manière détaillée en référence aux figures 3 à 6.

Dans ce premier mode de réalisation, l'accessoire vestimentaire 10 est un soutien-gorge comprenant une bande sous-poitrine 65 à partir de laquelle s'étend une première face 120 de la paroi latérale de la poche 20, ainsi que deux bonnets 125 placés de part et d'autre de la première face 120 et liés à la bande sous-poitrine 65, une deuxième face 130 de la poche 20, destinée à être en contact avec l'utilisateur, étant rapportée sur la première face 120.

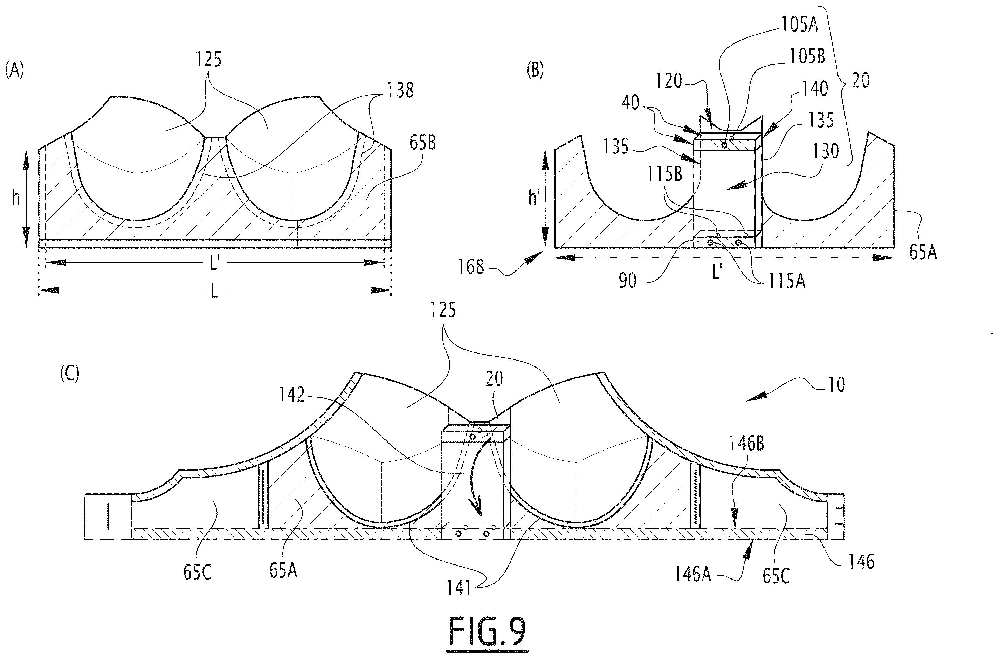

Dans un mode de réalisation particulier représenté sur la figure 9, la bande sous-poitrine 65 comprend :
- une pièce sous-poitrine avant interne 65A, dont une face est destinée à être en contact avec l'utilisateur et à laquelle est liée la poche 20,
- une pièce sous-poitrine avant externe 65B destinée à recevoir les bonnets 125 et à être visible de l'extérieur de l'utilisateur, une fois superposée au moins partiellement à la pièce sous-poitrine avant interne 65A à l'opposé de la deuxième face 130 de la poche 20, et
- deux parties dos 65C destinées à être attachées l'une à l'autre pour le maintien du soutien-gorge en place sur l'utilisateur.

Les zones de superposition des pièces sous-poitrine avant externe 65B et interne 65A sont représentées par des hachures larges sur les figures 9A et 9B.

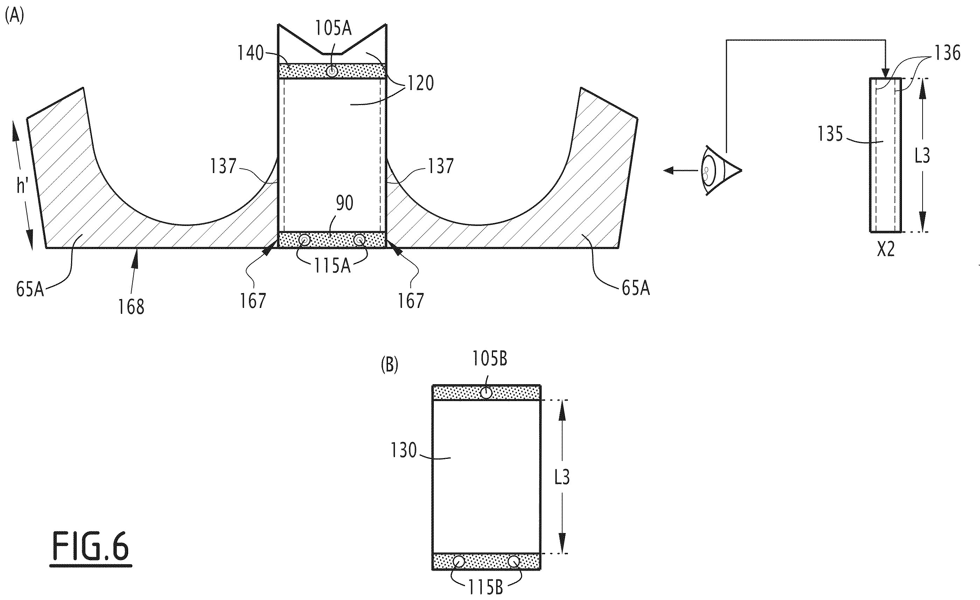

Dans ce cas, la première face 120 de la paroi latérale de la poche 20 s'étend à partir de la pièce sous-poitrine avant interne 65A, et est par exemple formée d'un seul tenant avec cette pièce sous-poitrine avant interne 65A, comme représenté sur les figures 6A et 9B.

Pour ce premier mode de réalisation, le soutien-gorge doit faire l'objet d'une fabrication spécifique, par exemple selon la méthode décrite ci-après.

Il n'est notamment pas possible de rapporter la poche 20 sur un soutien-gorge disponible dans le commerce. En effet, le séparateur (aussi appelé gousset ou en anglais, « center gore ») du soutien-gorge, qui relie les deux bonnets sur l'avant, ne présente pas dans un soutien-gorge du commerce une hauteur suffisante pour la fixation de la poche 20.

A titre d'exemple, la distance D entre le fond 90 de la poche 20 et le bord supérieur 100 doit typiquement être d'au moins 10 cm et la deuxième largeur L2 doit typiquement être supérieur à 5 cm.

La première face 120 est donc conçue spécifiquement pour à la fois jouer le rôle de séparateur et former une portion de la paroi latérale 85 de la poche 20.

Un procédé de fabrication du soutien-gorge selon l'invention avec armatures est décrit ci-après, dans le cas où la bande sous-poitrine 65 comprend la pièce sous-poitrine avant interne 65A et la pièce sous-poitrine avant externe 65B. L'homme du métier adaptera sans difficulté ce procédé à la fabrication, plus simple, d'un soutien-gorge sans armatures.

Ce procédé de fabrication comprend :
a) la fourniture des pièces du soutien-gorge comprenant, selon le patron de la figure 6 :
   * la bande sous-poitrine 65, notamment la pièce sous-poitrine avant interne 65A et la pièce sous-poitrine avant externe 65B et les parties dos 65C séparément,
   * les bonnets 125, deux armatures (non représentées) et deux cache-armature (non représentés) respectifs,
   * la première face 120, éventuellement d'un seul tenant avec la pièce sous-poitrine avant interne 65A, la deuxième face 130 et deux faces latérales 135 de la poche 20, les dimensions de ces pièces étant choisies en fonction de l'utilisateur et du dispositif médical 30 ;
b) l'assemblage des faces latérales 135 de part et d'autre de la première face 120 et de part et d'autre de la deuxième face 130 relativement à l'axe principal Z2, par exemple par couture à 0,5 cm du bord de la deuxième face 130 parallèlement à l'axe principal Z2 selon les traits de coutures 136 indiqués sur la partie A de la figure 6, de manière à former la poche 20 en laissant libres les première et deuxième faces 120, 130 au niveau d'une partie haute 140 et du fond 90. Une entaille 137, par exemple d'environ 1 cm, peut être réalisée de chaque côté à la jonction entre la première 120 et la pièce sous-poitrine avant interne 65A. Ces entailles permettent de glisser la pièce sous-poitrine avant interne 65A sous le cache-armature, cousu sur le haut de la marge de couture, de la pièce sous-poitrine avant externe 65B ;
c) l'assemblage des bonnets 125 avec la bande sous-poitrine 65, en particulier avec la pièce sous-poitrine avant externe 65B, par couture selon les traits de coutures 138 indiqués sur figure 9 partie A. Le résultat de cette deuxième étape d'assemblage c) est représenté sur la figure 9 partie A ;
d) la couture du haut des cache-armatures respectifs sur la marge de couture entre le bonnet 125 respectif et la pièce sous-poitrine avant externe 65B de la bande sous-poitrine 65 ;
e) le positionnement de la pièce sous-poitrine avant interne 65A assemblée obtenue à l'étape d'assemblage b) au-dessus de la pièce sous-poitrine avant externe 65B obtenue à l'étape d'assemblage c) de manière à ce que :
   - la poche 20 s'étende entre les bonnets 125, et
   - la deuxième face 130 de la poche 20 soit à l'opposé de la pièce sous-poitrine avant externe 65B relativement à la pièce sous-poitrine avant externe 65B, ou autrement dit de manière à ce que la poche 20 soit à l'intérieur du soutien-gorge relativement à l'utilisateur, comme représenté sur la figure 9 partie C,
   et l'insertion de la partie sous-poitrine de la poche 20 sous le cache-armature ;
f) la couture du bas, non encore cousu, de chaque cache-armature, selon les traits de couture 141 de la figure 9 partie C sur la pièce sous-poitrine avant externe 65B, en prenant en sandwich la pièce sous-poitrine avant interne 65A. Pour cette couture, on pourra abaisser le haut de la poche 20 en la repliant de haut en bas au-dessus de la pièce sous-poitrine avant interne 65A, selon la flèche 142 de la figure 9 partie C, de manière à ne pas coudre la poche 20 simultanément avec le cache-armature ;
g) la couture des parties dos 65C de la bande sous-poitrine 65 avec la pièce sous-poitrine avant externe 65A, comme visible sur la figure 9 partie C, en laissant une marge de couture par exemple de 0,7 mm à 1 cm ; puis le repliement de la marge de couture vers l'intérieur en prenant en sandwich la pièce sous-poitrine avant externe 65B, et la couture de l'ensemble avec d'éventuels cache-renforts latéraux ;
h) si nécessaire, la découpe des armatures pour qu'elles s'arrêtent au niveau de la poche 20 et leur insertion dans les cache-armature ;
i) la fermeture des cache-armature, en particulier à la limite de la poche 20, pour former les coutures intermédiaires 150A et 150B visibles sur la figure 5 ;
j) - la couture du bord bas 146A d'un élastique sous-poitrine 146 sur toute la longueur de la bande sous-poitrine 65 perpendiculairement à l'axe principal Z2, c'est-à-dire le long d'un bord inférieur des parties dos 65C et de la pièce sous-poitrine avant externe 65B, puis - la couture d'un bord supérieur 146B de élastique sous-poitrine 146 sur toute la longueur de la bande sous-poitrine, après repliement de bas en haut l'élastique sous-poitrine 146 vers la pièce sous-poitrine avant interne 65A de manière à prendre cette dernière en sandwich entre l'élastique sous-poitrine 146 et la pièce sous-poitrine avant externe 65B. On notera que l'étape de couture j) est effectuée sans que l'élastique sous-poitrine 146 ne soit cousu à la poche 20, grâce à la découpe sur la hauteur de l'élastique sous-poitrine146 formée dans la pièce sous-poitrine avant interne 65A de part et d'autre des côtés 167 du fond 90 de la poche 20. La pièce sous-poitrine avant interne 65A a de préférence une hauteur h' inférieure à celle de la pièce sous-poitrine avant externe 65B de manière à éviter de former une surépaisseur lors de cette étape. On constate que dans ce procédé, la continuité de l'élastique sous-poitrine 146 et son aptitude à se déformer est préservée malgré la présence de la poche 20. Le confort pour l'utilisateur est donc optimal ;
k) la couture d'une partie haute 140 de la poche 20 avec les bonnets 125, en suivant la courbe du haut de chaque bonnet 125, pour former par exemple les coutures hautes 140A et 140B visibles sur la figure 5, de préférence seulement au niveau de la première face 120;
l) l'assemblage d'éventuelles bretelles 70, attaches et élastiques sous-bras, élastiques dos, et élastiques de décoration, notamment en suivant la courbe des bonnets 125 en partie haute au niveau de la première face 120 ;
m) l'assemblage du dispositif de fermeture principal 115, et le cas échéant du dispositif de fermeture auxiliaire 105 avec les première et deuxième faces 120, 130, si cela n'a pas été déjà fait.

La réalisation de la bande sous-poitrine 65 au moyen des pièces sous-poitrine avant externe et interne 65B, 65A permet de rendre la poche 20 la plus indépendante possible du reste du soutien-gorge, de manière à assurer un positionnement et un assemblage aisé des différents éléments du soutien-gorge lors de sa fabrication, mais aussi à assurer un excellent confort pour l'utilisateur.

Notamment la portion de la deuxième face 130 qui se trouve entre le fond 90 et la partie haute 90 peut ne pas être cousue directement à la pièce sous-poitrine avant externe 65B, mais lui être liée seulement par l'intermédiaire des faces latérales 135.

La poche 20 peut donc se déformer sans provoquer de déformation substantielle de la pièce sous-poitrine avant externe 65B, ni modification significative de la tension de l'élastique sous-poitrine 146.

Le déploiement de la poche 20 pour accueillir le boîtier 25 est alors particulièrement souple et n'impacte que peu la souplesse du soutien-gorge pour assurer sa fonction première de maintien confortable de la poitrine.

La formation de la première face 120 et de la pièce sous-poitrine avant interne 65A de la bande sous-poitrine 65 d'un seul tenant, comme représenté sur la figure 6, permet une meilleure stabilité du boîtier 25 inséré dans la poche 20.

De manière générale, la partie haute de la première face 120 de la poche 20 est à adapter suivant la forme du haut des bonnets 125.

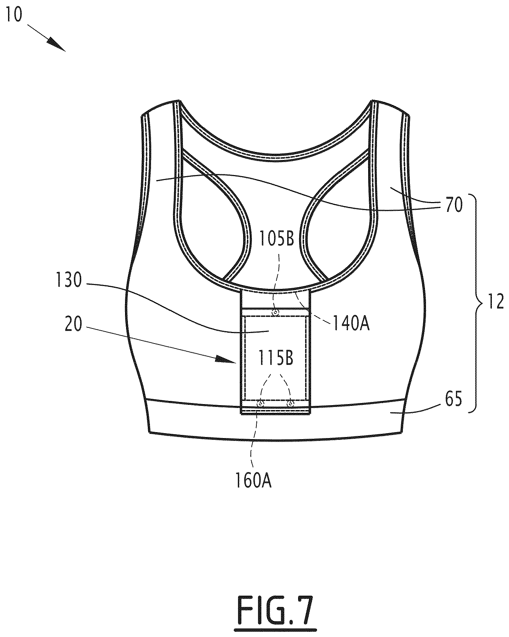

L'accessoire vestimentaire 10 selon un deuxième mode de réalisation va maintenant être décrit en référence à la figure 7.

Ce deuxième mode de réalisation diffère du premier mode de réalisation en ce que l'accessoire vestimentaire 10 est une brassière.

Dans ce cas, l'accessoire vestimentaire est particulièrement adapté pour être porté pour faire du sport ou pour dormir.

Dans le cas d'une utilisation pour dormir, on pourra en particulier former la brassière avec un élastique de maintien et un tissu élastique plus souples que pour une utilisation pour la pratique du sport, de manière à ce que la tension et le maintien soient moins forts que pour la brassière sport et que le port de la brassière soit ainsi particulièrement confortable, tout en conservant à la brassière une tenue suffisante pour supporter le dispositif médical 30.

Dans ce deuxième mode de réalisation, la poche 20 est rapportée sur une face interne d'un devant d'une brassière conventionnelle.

Dans ce cas, l'accessoire vestimentaire 10 peut être fabriqué de toute pièce, par simple adaptation de la méthode décrite ci-avant.

Alternativement, l'accessoire vestimentaire 10 peut être fabriqué à partir d'un kit de fabrication comprenant une brassière conventionnelle, disponible dans le commerce, et une poche 20 selon l'invention.

La poche 20 est assemblée à la brassière standard par couture de la poche 20 au moins pour former les coutures hautes 140A,140B et basses par exemple de la même manière que pour le premier mode de réalisation ou encore entre la brassière et respectivement une partie haute et une partie basse de la première face 120 débordant relativement à la deuxième face 130 selon l'axe principal Z2, comme visible sur la figure 7.

Encore alternativement, l'accessoire vestimentaire 10 peut être fabriqué à partir d'un kit de fabrication comprenant une brassière standard, disponible dans le commerce, et les pièces permettant de former une poche 20 selon l'invention selon l'étape b) de la méthode de fabrication d'un soutien-gorge avec armatures décrite plus haut.

On comprend que dans le deuxième mode de réalisation comme dans le premier mode de réalisation, la poche 20 peut n'être cousue au dispositif de maintien 12, qui comprend dans ces exemples la bande sous-poitrine 65 et éventuellement les bretelles 70, qu'au niveau de la partie haute et de la partie basse de la première face 120, ce qui rend l'accessoire vestimentaire 10 particulièrement confortable.

D'autres modes de réalisation pourront être envisagés par l'homme du métier sans difficulté.

A titre d'exemple, le dispositif de maintien 12 de l'accessoire vestimentaire 10 peut se présenter sous la forme d'un tee-shirt ou d'une blouse, sur une surface intérieure duquel ou de laquelle est positionnée la poche 20.

Le dispositif de maintien 12 peut encore se présenter sous la forme d'un harnais, notamment pour un enfant.

Quel que soit le mode de réalisation, l'accessoire vestimentaire 10 selon l'invention permet de maintenir le boîtier 25 du dispositif médical sur le corps 15 de l'utilisateur de manière confortable, tout en laissant à l'utilisateur la possibilité d'interagir à volonté avec l'interface utilisateur 45 et en limitant les risques de repliement, pincement ou arrachage de la tubulure 40 par rapport aux accessoires vestimentaires de l'art antérieur.

Le boîtier 25 est de plus porté de manière discrète, peu visible pour des observateurs de l'utilisateur, lorsque la poche 20 est placée à l'intérieur de l'accessoire vestimentaire.

Les exemples permettent notamment de comprendre que les coutures de la poche 20 peuvent ne pas être apparentes sur l'extérieur de l'accessoire vestimentaire 10.

Dans le cas où l'accessoire vestimentaire 10 est porté sur le buste 17, la majorité des sites d'insertion 60 de la canule 55 privilégiés pour le traitement du diabète peuvent être exploités.

Dans ce même cas, la poche 20 peut être positionnée dans une zone entre-seins de sorte que le port du boîtier 25 est particulièrement discret et confortable, notamment sans risque de descente du boîtier vers l'estomac.

Dans le cas d'un accessoire vestimentaire 10 de type soutien-gorge ou brassière, l'habillage est particulièrement facile une fois l'accessoire vestimentaire 10 en place et le dispositif médical 30 en place, la tubulure 40 étant naturellement bien positionnée grâce à l'accessoire vestimentaire 10.

## Revendications

1. Accessoire vestimentaire (10) comprenant un dispositif de maintien (12) sur le corps (15), notamment sur le buste (17), d'un utilisateur et une poche (20) de réception d'un boîtier (25) d'un dispositif médical (30) d'injection de liquide, le boîtier (25) comprenant un réservoir (35) de liquide connecté à une tubulure (40) du dispositif médical (30) s'étendant hors du boîtier (25),
la poche (20) étant solidaire du dispositif de maintien (12),
la poche (20) présentant une paroi latérale (85) s'étendant selon un axe principal (Z2) entre un fond (90) et une ouverture supérieure (95) destinée à être positionné plus haut que le fond (90) selon un axe longitudinal (Z1) d'un utilisateur orienté des pieds vers la tête de cet utilisateur, l'ouverture supérieure (95) étant configurée pour permettre l'extraction du boîtier (25) hors de la poche (20) par translation selon l'axe principal (Z2) du fond (90) vers l'ouverture supérieure (95),
l'accessoire vestimentaire (10) étant **caractérisé en ce qu'**il est de type soutien-gorge ou brassière,
**en ce que** le fond (90) comprend une ouverture inférieure (110) et un dispositif de fermeture principal (115) de l'ouverture inférieure (110) présentant :
- au moins une position ouverte, permettant l'insertion du boîtier (25) dans la poche (20) par translation selon l'axe principal (Z2) de l'ouverture inférieure (110) vers l'ouverture supérieure (95), et
- au moins une position fermée, empêchant le passage du boîtier (25) au travers de l'ouverture inférieure (110) tout en permettant la translation de la tubulure (40) selon l'axe principal (Z2),
et **en ce que** le dispositif de maintien (12) comprend une bande sous-poitrine (65) de laquelle le fond (90) de la poche (20) est solidaire.

2. Accessoire vestimentaire (10) selon la revendication 1, dans lequel l'ouverture supérieure (95) et l'ouverture inférieure (110) sont espacées selon l'axe principal (Z2) d'une distance supérieure ou égale à 90% d'une hauteur (H1) du boîtier (25) du dispositif médical (30) d'injection de liquide entre une face (25A) du boîtier (25) sur laquelle est montée un connecteur (50) de la tubulure (40) et une face opposée (25B) du boîtier (25).

3. Accessoire vestimentaire (10) selon l'une quelconque des revendications précédentes, comprenant en outre un dispositif de fermeture auxiliaire (105) de l'ouverture supérieure (95) présentant :
- au moins une position ouverte, permettant l'extraction du boîtier (25) hors de la poche (20) par translation selon l'axe principal (Z2) du fond (90) vers l'ouverture supérieure (95), et
- au moins une position fermée, empêchant le passage du boîtier (25) au travers de l'ouverture supérieure (95).

4. Accessoire vestimentaire (10) selon l'une quelconque des revendications précédentes, dans lequel le dispositif de fermeture principal (115) et/ou, le cas échéant, le dispositif de fermeture auxiliaire (105) est choisi parmi un fermoir à pression, un fermoir magnétique, un fermoir à agrafe et une fermeture à glissière.

5. Accessoire vestimentaire (10) selon l'une quelconque des revendications précédentes, dans lequel la poche s'étend au-dessus de la bande sous-poitrine (65) dans une portion entre-seins.

6. Accessoire vestimentaire (10) selon l'une quelconque des revendications 1 à 5, de type soutien-gorge, dans lequel une première face (120) de la paroi latérale (85) de la poche (20) s'étend à partir de la bande sous-poitrine (65), deux bonnets (125) étant placés de part et d'autre de la première face (120) et liés à la bande sous-poitrine (65), une deuxième face (130) de la poche (20), destinée à être en contact avec l'utilisateur, étant rapportée sur la première face (120).

7. Accessoire vestimentaire (10) selon la revendication 6, dans lequel :
- la première face (120) s'étend à partir de ou est formée dans une pièce sous-poitrine avant interne (65A) de la bande sous-poitrine (65) rapportée sur une pièce sous-poitrine avant externe (65B) de la bande sous-poitrine (65), et
- l'accessoire vestimentaire (10) comprend un élastique sous-poitrine (146) lié à la pièce sous-poitrine avant externe (65B) s'étendant sur toute la longueur de la bande sous-poitrine (65) perpendiculairement à l'axe principal (Z2).

8. Accessoire vestimentaire (10) selon l'une quelconque des revendications 1 à 5, de type brassière, dans lequel la poche est rapportée sur une face interne d'un devant d'une brassière conventionnelle.
